# EUROPEAN PATENT APPLICATION

(11) **EP 3 299 454 A1**
(43) Date of publication of application: **28.03.2018**
(21) Application number: 16189921.6
(22) Date of filing: 21.09.2016
(51) Int. Cl.: C12N 7/00, C12N 5/00

(54) **OPTIMIZED METHOD FOR LARGE SCALE PRODUCTION OF PARVOVIRUS H-1 IN AN ESSENTIALLY SERUM-FREE MEDIUM**

(71) Applicant: Deutsches Krebsforschungszentrum, 69120 Heidelberg (DE)
(72) Inventor: LEUCHS, Barbara, 69121 Heidelberg (DE); ROMMELAERE, Jean, 69118 Heidelberg (DE); Lutz, Sebastian, 41836 Hückelhoven (DE); Vogel, Martin, 67117 Limburgerhof (DE)
(74) Representative: Schüssler, Andrea

(57) **Abstract**

Described is an optimized process for parvovirus production including an essentially serum-free medium which is suitable to increase parvovirus production compared to a standard medium, preferably for H-1 PV production.

## Description

### Field of the invention

The present invention provides a process for parvovirus production using an essentially serum-free medium which is suitable to increase parvovirus production compared to a standard medium.

### Background of the invention

H-1PV belongs to the genus Protoparvovirus within the Parvovirinae subfamily of Parvoviridae (Cotmore et al., 2014). It consists of a non-enveloped icosahedral capsid 25 nm in diameter and contains a single-stranded DNA genome about 5 kb long, encoding non-structural proteins - notably NS1 (83 kDa) and NS2 (25 kDa) - and the capsid proteins VP1 (81 kDa) and VP2 (65 kDa). Another capsid protein, VP3 (63 kDa), is generated by post-translational cleavage of VP2 (Faisst et al., 1995; Halder et al., 2012; Hanson and Rhode, 1991; Toolan et al., 1960). Protoparvoviruses replicate in a S-phase-dependent fashion and undergo a lytic cycle after infection of permissive cells (Burnett et al., 2006).

While the natural host of H-1 PV is the rat, this virus has recently raised much interest because it replicates preferentially in transformed cells, including a number of human tumor cells. The virus thus has oncolytic and oncosuppressive properties that have been demonstrated in various cell cultures and animal models (Nuesch et al., 2012; Rommelaere et al., 2010). In xenograft models, H-1 PV has been shown to suppress a number of human tumors, including cervical tumors (Faisst et al., 1998; Li et al., 2013), pancreatic tumors (Angelova et al., 2009b; Grekova et al., 2011), mammary carcinomas (Dupressoir et al., 1989), gliomas (Geletneky et al., 2010; Kiprianova et al., 2011), and lymphomas (Angelova et al., 2009a). In addition, H-1 PV has been shown to be successful in eliminating cancer stem cells (EP 2 404 609 A1). On the basis of these preclinical proofs of concept, a first clinical trial (phase I/IIa) of H-1 PV was launched in 2011, for patients with recurrent glioblastoma multiforme (Geletneky et al., 2012).

To test and eventually exploit the therapeutic potential of H-1 PV, it is necessary to optimize the production of purified virus (vector) stocks in medium suitable for preclinical or clinical applications or basic research.

However, cell culture media formulations have been well documented in the literature and a number of media are commercially available. H-1 PV production is routinely carried out in various cell cultures, such as human NB-324K cells, using standard cell culture media.

The requirements of human cell culture *in vitro* comprise, in addition to basic nutritional substances, a complex series of growth factors (Werner et al., 1993). The supplementation of standard cell culture media with animal serum is essential for cell growth, metabolism, and to stimulate proliferation. The sera used most widely are bovine sera of adult or newborn animals, or of fetal origin in a range of 10-20% v/v. Fetal bovine serum (FBS) is a cocktail of most of the factors required for cell attachment, growth and proliferation and is thus used as an almost universal growth supplement effective for most types of human and animal cells. However, the use of animal serum in cell culture also bears a number of disadvantages. These disadvantages can be seen either from a cell biological point of view, since serum in general is an ill-defined mixture of components in culture media. Also, for the biotechnological production of human therapeutics, animal-derived components cannot be applied in culture protocols. For example, there is a risk that the culture medium or products purified from it may be immunogenic, particularly if the supplements are derived from an animal different from the source of the cells to be cultured and induce an immunological reaction.

A number of serum-free media formulations are commercially available, such as those designed to support the culture of endothelial cells. Serum-free media are lower in protein content than medium supplemented with serum, which can simplify the purification process, increase the yield of the end-product, introduce improved control of cell culture conditions, increased lot-to-lot consistency, and optimized formulations for specific cell types. Further, characteristics and compositions of the cell culture media vary depending on the particular cellular requirements. Important parameters include osmolarity, pH, and nutrient formulations.

To overcome the limitations of the use of animal proteins in serum-free media several attempts have been made to construct animal cell culture media that are completely free of animal proteins. For example, some culture media have incorporated extracts of yeast cells into the basal medium (U.K. Patent Application No. GB 901673; Keay, L., Biotechnol. Bioengin. 17:745-764 (1975)) to provide sources of nitrogen and other essential nutrients.

However, none of these approaches provided a culture medium that is optimal for the cultivation of non-commercial NB-324K cells for the large scale production of H1-PV for preclinical or clinical applications. Since NB-324K cells are generally difficult to grow, it is not possible to just adapt media known from the cultivation of other commercial organisms.

Thus, there remains a need for an essentially serum-free culture medium which facilitates the growth of NB-324K cells to increase the productivity of H-1 PV. Such culture media should allow easier and more cost-effective production and purification of the parvovirus produced by cultured NB-324K cells and will provide more consistent results.

Thus, the technical problem underlying the present invention is to optimize the parvovirus large scale production in an essentially serum-free medium.

The solution of said technical problem is achieved by providing the embodiments characterized in the claims.

### Summary of the invention

Oncolytic protoparvovirus research has reached the stage of translation into clinical practice, with a first phase I/IIa study of H-1PV in patients with recurrent resectable malignant glioma (Geletneky et al., 2012). This trial is expected to be followed by further clinical studies aiming to assess efficacy and to extend the approach to other cancers such as pancreatic carcinoma (clinical trial: "ParvOryx02"; https://clinicaltrials.gov/ct2/show/NCT02653313) or neuroblastoma (Lacroix et al., 2010; Li et al., 2013). These developments rely on the availability of robust procedures for protoparvovirus production in a large scale.

During the experiments resulting in the present invention major innovations for optimization of the H1-PV production, with elimination of unwanted contaminants in cell culture media through FBS were introduced. The inventors focused on developing an essentially serum-free medium as a basis for the production of H-1 PV for preclinical and clinical applications in the anticancer virotherapy.

The process of the present invention uses an essentially serum-free cell culture medium comprising the ingredients glucose, glutamine, glutamate, lactate, ammonium, proteins (e.g. growth factors, insulin, etc.), wherein the medium is capable of supporting the cultivation of epithelial cells *in vitro,* preferably NB-324K cells, for the production of H-1 PV.

### Brief description of the drawings

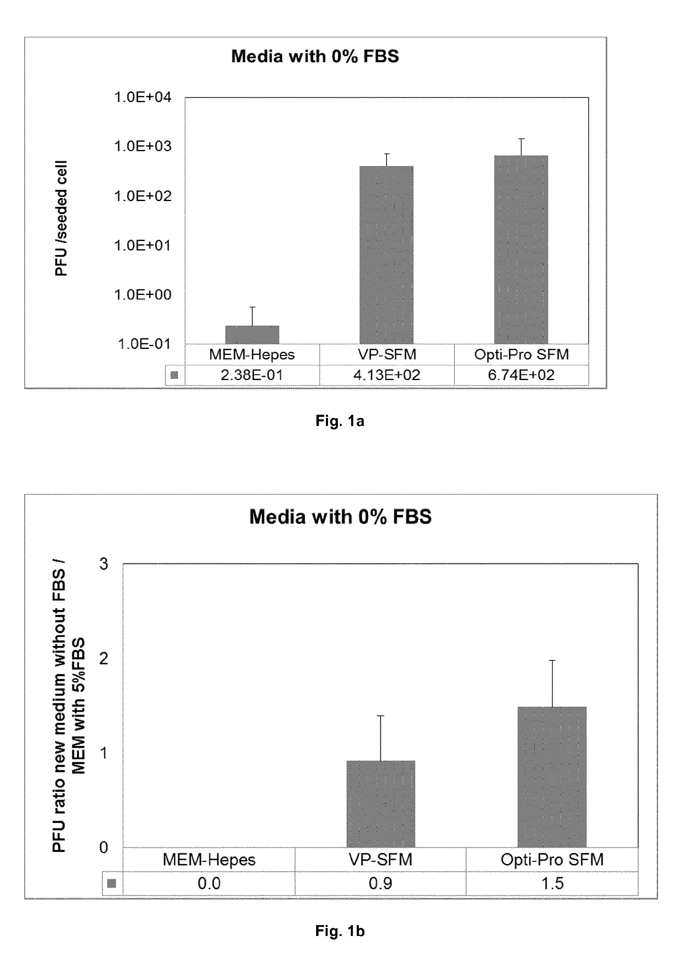

Figure 1a and 1b: Different media for higher H1-PV productivity without FBS
   Fig. 1 a depicts a bar graph showing the effect of MEM-Hepes, VP-SFM and Opti-Pro SFM media on the H-1 PV production in NB-324K cells without FBS. The PFU titers were determined via Plaque-Formation-Assay and are expressed relative to the number of seeded cells.
   Fig. 1b depicts a bar graph showing the effect of MEM-Hepes, VP-SFM and Opti-Pro SFM media on the H-1 PV production in NB-324K cells without FBS. PFU ratio of VP-SFM and Opti-Pro SFM without FBS was expressed relative to MEM-Hepes medium with 5% FBS.
Figure 2a and 2b: Different media for higher H1-PV productivity with 5% FBS

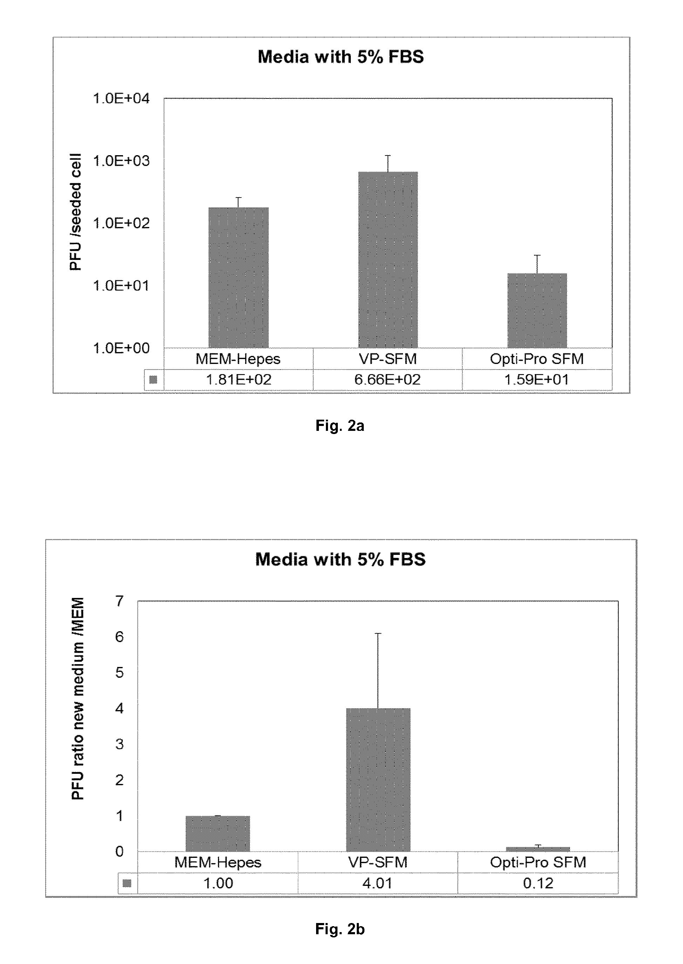

   Fig. 2a depicts a bar graph showing the effect of MEM-Hepes, VP-SFM and Opti-Pro SFM media on the H-1 PV production in NB-324K cells with 5% FBS. The PFU titers were determined via Plaque-Formation-Assay and are expressed relative to the number of seeded cells.
   Fig. 2b depicts a bar graph showing the effect of MEM-Hepes, VP-SFM and Opti-Pro SFM media on the H-1PV production in NB-324K cells with 5% FBS. PFU ratio of VP-SFM and Opti-Pro SFM with 5% FBS was expressed relative to MEM-Hepes medium with 5% FBS.

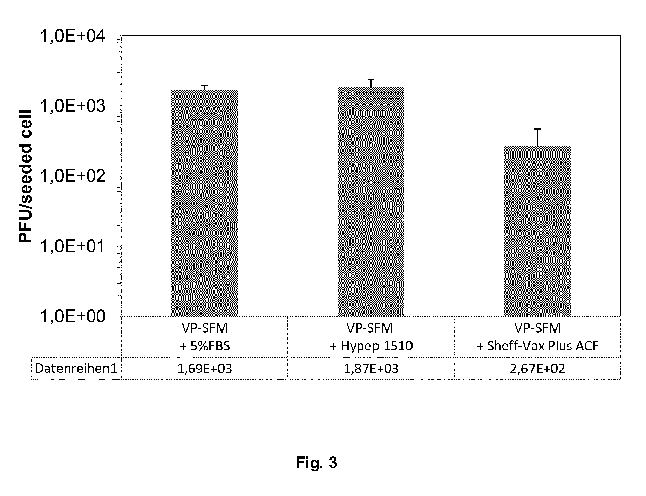

Figure 3: VP-SFM medium with and without FBS supplemented with Hypep 1510 or Sheff-Vax Plus ACF
   Fig. 3 depicts a bar graph showing the effect of VP-SFM supplemented with 5% FBS, VP-SFM supplemented with Hypep 1510 and VP-SFM supplemented with Sheff-Vax Plus ACF on the H-1 PV production in NB-324K cells. The PFU titers were determined via Plaque-Formation-Assay and are expressed relative to the number of seeded cells.

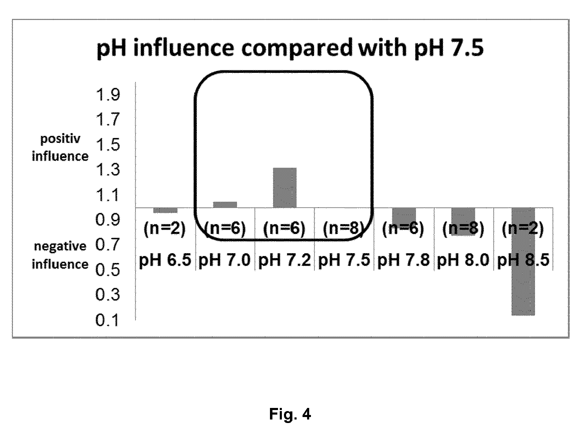

Figure 4: pH influence of H-1PV productivity
   Fig.4 depicts a bar graph showing the effect of pH of 6.5, 7.0, 7.2, 7.5, 7.8, 8.0 and 8.5 adjusted in MEM-Hepes medium either with HCl or NaOH on the H-1 PV production in NB-234K cells. The PFU titers were determined via Plaque-Formation-Assay.

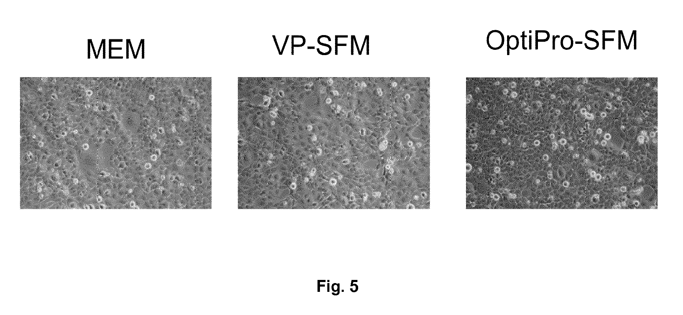

Figure 5: Morphology of NB-234K in different media
   Fig.5 depicts the morphology of NB-324K cells in MEM-Hepes, VP-SFM and Opti-Pro SFM media via light microscopy.

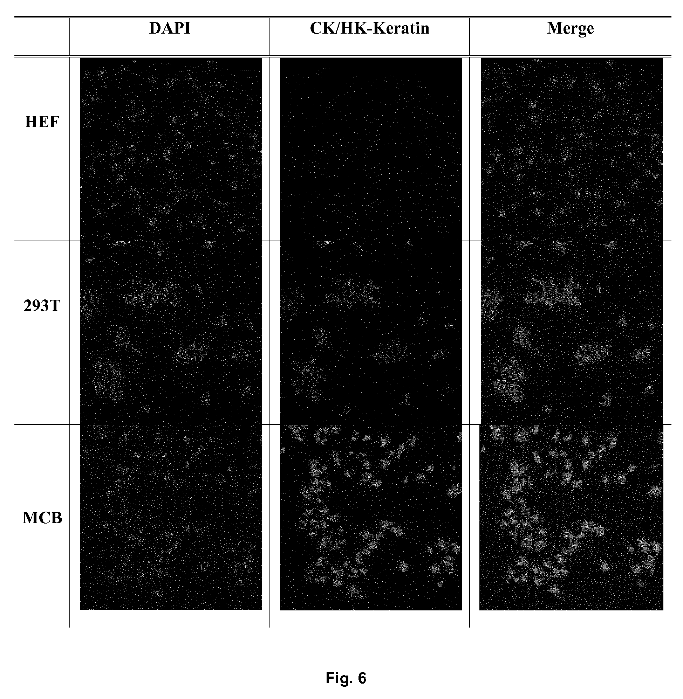

Figure 6: Immunofluorescence Staining
   Fig. 6 shows that the master cell line (MCB) is an epithelial cell line like the compared 293 T human embryonic kidney cell line and differs from a fibroblast cell line (HEF).

### Detailed description of the invention

The term "cell culture" means the maintenance of cells in an artificial, *in vitro* environment. The media of the present invention can be used to culture adherent mammalian cell, i.e. a cell which adheres to the culture vessel, preferably epithelial cells like NB-324K cells.

The term "cultivation" means the maintenance of cells *in vitro* under conditions favoring growth, differentiation or continued viability, in an active or quiescent state, of the cells.

The phrase "cell culture medium" refers to a nutritive solution for cultivating cells.

The term "EGF" refers to an epidermal growth factor that stimulates proliferation and differentiation by binding to its receptor EGFR. Further, EGF regulates migration and cell growth of intestinal epithelial cells. The EGF is preferably human EGF (hEGF).

The term "ingredient" refers to any compound, whether of chemical or biological origin, that can be used in cell culture media to maintain or promote the growth of proliferation of cells. The terms "component," "nutrient" and ingredient" can be used interchangeably and are all meant to refer to such compounds. Typical ingredients that are used in cell culture media include glucose, glutamine, glutamate, lactate, ammonium, growth factors, insulin and proteins.

A "serum-free" medium is one which contains no serum. A serum-free medium is distinguished from low-serum and essentially serum-free media, both of which contain serum.

The invention relates to a process for producing parvovirus H-1 by growing H-1 infected producer cell line NB-324K ("the master cell bank" or "master cell line") in a serum-free culture medium, wherein the medium comprises the below mentioned ingredients. As shown in the Example part the present invention produces a four-fold higher parvovirus titer than usually achieved with a standard FBS-containing medium.

The process of the present invention uses an serum-free cell culture medium comprising the ingredients glucose, glutamine, glutamate, lactate, ammonium, proteins (e.g. growth factors, insulin, etc.), wherein the medium is capable of supporting the cultivation of epithelial cells *in vitro,* preferably NB-324K cells, for the production of H-1 PV.

The preferred growth factor used in the media of the invention is epithelial growth factor (EGF), preferably human EGF (hEGF), while the preferred insulin is human recombinant. The protein ingredients of the present media have no human or animal origin and the protein concentration is ultra low, preferably less than 10µg/µl.

The medium of the present invention can be used to grow human epithelial cells, preferably NB-324K cells, to high density and/or to increase parvovirus production. In one preferred embodiment, the process of the present invention uses an essentially serum-free cell culture medium containing about 16-22 mM glucose, 3-5 mM glutamine, 0.1-0.6 mM glutamate, 0.5-1.0 mM lactate, less than 0.3 mM ammonium and 3-10 µg/µl proteins , wherein the medium is capable of supporting the cultivation of NB-324K cells for the production of H-1 PV.

Preferably, the culture.medium contains 17-20 mM glucose, about 4mM glutamine, about 0.15-0.5 mM glutamate, about 0.7 mM lactate, less than 0.2 mM ammonium and 4-8 µg/µl proteins. (e.g. supplements, epithelial growth factor and insulin).

In a particular preferred embodiment the culture medium contains 19.14 mM glucose, 4.25 mM glutamine, 0.174 mM glutamate, 0.669 mM lactate, less than 0.05 mM ammonium and about 5 µg/µl proteins. These proteins include EGF, insulin and proteineous supplements. This medium is called "VP-SFM".

In another preferred embodiment, the culture medium contains 17.49 mM glucose, 4.25 mM glutamine, 0.486 mM glutamate, 0.641 mM lactate, 0.18 mM ammonium, 7.5 ng/µl rEGF and about 7.5 µg/µl proteins. These proteins include EGF and proteineous supplements. This medium is called "Opti-Pro SFM".

Supplements which may be added to the present media include insulin, soy peptides (e.g. Hypep 1510), Sheff-Vax Plus ACF, Insulin-Transferrin-Selenium A, Insulin-Transferrin-Seelenium X and CD Lipid Concentrate. Insulin is available commercially, for example from Life Technologies, Inc. (Rockville, Md.). Soy peptides are commercially available, for example from Quest International (Norwich, N.Y.).

In a preferred embodiment, the medium of the present invention is combined with a supplement, preferably soy proteins. One suitable soy protein is ultrafiltered enzymatic digest of soy, e.g. Hypep 1510® (Kerry Ingredients & Favors, Norwich, NY, USA). Another suitable supplement is Sheff-Vax Plus ACF® (Kerry, Inc., Kildire, Ireland) which is a vegetable component-based hydrolysate that contains growth factors and trace elements.

In further preferred embodiment, the pH of the medium of the present invention should be adjusted to around 7.0-7.5, preferably about 7.2.

The medium of the present invention may be used to facilitate cultivation of epithelial cells. In particular, these media may be used to cultivate the master cell line based on NB-324K cells for which the inventors found out that it is an epithelial cell line. While the present medium is particularly useful for culturing epithelial cells, it is understood that the medium may be used in any standard cell culture protocol whether the cells are grown in a method by which epithelial cells may be cultivated *in vitro.*

Furthermore, since the present medium is serum-free and has a low-protein concentration, the medium may be used for rapid production of a parvovirus, e.g.H-1 PV. In the present application it has been demonstrated that the production of H1-PV in VP-SFM supplemented with glutamine and soy peptides showed the same yield compared to a FBS-containing medium and are a suitable alternative for the serum-free production. Another suitable medium is Opti-Pro SFM supplemented with glutamine.

The following examples are intended to illustrate, but not to limit the invention. While such examples are typical of those that might be used, other methods known to those skilled in the art may alternatively be utilized.

### Example 1

### Material and Methods

### (A) Medium for cell cultivation and H-1PV production

The MEM medium was supplemented with 2mM L-Glutamin. VP-SFM and OptiPro-SFM were each supplemented with 4mM L-Glutamin.

| Medium | Information | Additive |
|---|---|---|
| MEM | Basal medium + HEPES, pH mit HCl, 2mM L-Glutamin; 1,8mM CaCl₂; 0,814mM MgSO₄, pH to 7,6-7,7 adjusted | 2mM L-Glutamin |
| VP-SFM (GIBCO Invitrogen, Paisley, UK) | Serum free + ultra low protein 5µg/ml (no proteins/peptides of human/animal origin) - proteins: human recombinant EGF + human recombinant insulin, contains 3,9g/l glucose, contains no L-Gln; | 4mM L-Glutamin (=VP-SFM *) |
| OptiPRO SFM (GIBCO Invitrogen, Paisley, UK) | Serum-free + ultra low protein 7,5µg/ml, contains 7,5ng/ml rEGF, contains no L-Gln | 4mM L-Glutamin (=Opti-Pro SFM*) |

### (B) Cell cultivation and H-1 PV production

NB-324K human newborn kidney cells (master cell bank) transformed with simian virus 40 (SV40) (Tattersall and Bratton, 1983) were cultured at 37°C in different medium (see above).

After transferring MCB cells to each medium the cells have been adapted to the cells at least for 2 weeks. For production, NB-324K cells were seeded at 3.6x10⁴ cells/cm² and infected immediately with H-1 PV at a multiplicity of infection (MOI) of 0.01 plaque forming units (PFU) per cell. The infected cells were incubated for 4 days at 37°C under 5% CO₂ until the cytopathic effect (CPE), measured as the percentage of detached cells observed under a microscope, reached at least 30%. Cell density and viability were measured by staining living cells with 0.4% trypan blue (Invitrogen™, Germany). Cells were counted with a Countess^{®} Cell counter (Life Technologies, Germany) and morphology is microscopical observed.

For harvesting, the medium was aspirated and infected cells were treated with PBS/1 mM EDTA. The medium supernatant and detached cells were centrifuged for 5 min at 5,000xg. The pellet was washed with PBS, resuspended in Virus Tris/EDTA buffer, pH 8.7 (VTE) containing 0.05 M Tris HCl, 0.5 mM EDTA, and subjected to three freeze/thaw cycles. After centrifugation for 5 min at 5,000xg, cell debris were discarded. The cell lysate was then sonicated at 48 W for 1 min in a Sonorex Super 10 P ultrasonic homogenizer (Bandelin, Germany) and treated with DNAse (50 U/ml, Sigma, Germany) for 30 min at 37°C.

### (C) Plaque formation assay (PFU)

Plaque assays were done essentially as described by Tattersall and Bratton, 1983. NB-324K cells were grown in monolayer cultures in MEM medium containing 5% FBS, 100 µg/ml penicillin, 100 µg/ml streptomycin, and 2 mM L-glutamine. They were infected at 60% confluence with serial dilutions of H-1 PV and incubated for 1 h at 37°C. Then the inoculum was replaced with a bacto-agar overlay (1.7% in MEM containing 5% FBS). On day four post-infection, living cells were stained for 18-24 h by addition of 0.02% toluylene red staining solution (Sigma, Germany) containing bacto-agar (Becton Dickinson, Germany). The dishes were incubated at 37°C under 5% CO₂. Plaque-forming units were counted 5 days post-infection on a light box and their concentration expressed in PFU/ml.

### (D) Immune fluorescence test

The immune fluorescence test is based on the use of fluorescence-marked antibodies which bind to her specific antigen by which certain extra and intracellular structures can be proved. This principle is used for keratin staining with a CH/HK-Keratin antibody to for of epithelial cells evidence. Therefore the cells were cultivated on slides in 145 cm² cell culture flasks (Greiner Bio-One). NB-324K human newborn kidney cells (master cell bank) were seeded with a density of 3E6 cells/flask. For the Keratin staining HEF (human embryonic fibroblasts) and HEK293T (human epithelial kidney cells) with a cell density of 1.2 E6 cells/flask or 5E6 cells were seeded as controls. After 24-hour incubation time the cells on the slides were washed in PBS, fixed for 10 minutes in methanol and afterwards 5 minutes in acetone. Both liquids were precooled before in -20°C. For the Keratin detection the slides were pre-blocked with PBS with 0. 05% BSA and afterwards incubated with the CH/HK-Keratin antibody for 1 hour. Afterwards slides were washed in PBS with 0. 1% Triton, before the second antibody Cy3gp (Dianova) was incubated for 1 hour. The unbound second antibodies were removed by triple washing in PBS with 0. 1% Triton. The slides were dipped in Ethanol and were dried. The slides were embedded with slide mounting medium (CELL LAB) including 1µg/ml DAPI, that stains the nucleus of the cells. The analysis occurred with the fluorescence microscope BZ-9000 (Keyence).

### (E) Microscopy of cells

With the light microscope (Axiovert 25) the cell morphology, density and shape is observed.

### Example 2

### Comparison of H-1 PV productivity of MEM-Hepes, VP-SFM and Opti-Pro SFM media without FBS

The inventors first determined the productivity of H-1 PV in different media including MEM-Hepes, VP-SFM supplemented with glutamine (VP-SFM*) and Opti-Pro SFM supplemented with glutamine (Opti-Pro SFM*) media, each without FBS (fetal bovine serum). The cells were harvested as described above and the infectious particles have been analyzed via PFU Assay.

As shown in Fig. 1a, H-1PV-infected NB-324K cells cultured in the VP-SFM* and Opti-Pro SFM* media with 0% FBS produced higher titers of H-1PV compared to standard medium MEM-Hepes with 0% FBS.

These results demonstrate that the VP-SFM* and Opti-Pro SFM* media without FBS facilitate the rapid production of H-1 PV compared to standard medium MEM-Hepes without FBS (see Fig. 1 a). VP-SFM* with 0% FBS (0.9 ± 0.5) and Opti-Pro SFM* (1.5 ± 0.5) with 0% FCS showed the same or even an increase in productivity compared to MEM-Hepes 5% FBS (comparison of Fig. 2b with Fig. 1b). Further, standard medium MEM-Hepes with 0% FBS showed a substantial decrease in productivity or almost no productivity (Fig. 1a).

VP-SFM" and Opti-Pro SFM* differ by the presence of EGF from the standard medium MEM-Hepes. This shows that the growth factor EGF could stimulate cell proliferation (Fallon et al., 1984) and this may facilitate the H1-PV productivity.

### Example 3

### Comparison of H-1 PV productivity in MEM-Hepes, VP-SFM and Opti-Pro SFM media with 5% FBS

To examine the H-1 PV productivity in MEM-Hepes, VP-SFM* and Opti-Pro SFM* media with each 5% FBS, NB-324K cells were harvested as described above and the infectious particles have been analyzed via PFU Assay.

As shown in Fig. 2b, VP-SFM* (+5% FBS) was about 4.0x ±2.3 better in H-1 PV productivity than the comparison-media MEM-Hepes (+5% FBS). Opti-Pro SFM* (+5% FBS) medium shows a lower PFU value than the comparison-medium MEM-Hepes (+5% FBS) (about 10% of MEM-Hepes).

These results demonstrate that Opti-Pro SFM* has a better productivity without the addition of FBS in comparison with added 5% FBS (Fig. 1 a and Fig. 2a). In contrast, standard medium MEM-Hepes showed a much better productivity with 5% FBS in comparison without FBS (Fig. 1 a and Fig. 2a).

### Example 4

### Measurement of the H-1 PV productivity in VP-SFM medium with Hypep 1510 or Sheff-Vax Plus ACF supplemented and without FBS

Hypep 1510® and Sheff-Vax Plus ACF® were used as additives in VP-SFM to replace FBS. The cells were cultured with 5 % FBS in VP-SFM and in serum-free VP-SFM supplemented with Hypep 1510® or Sheff -Vax Plus ACF ®. The cells were harvested as described above and the infectious particles have been analyzed via PFU Assay.

As shown in Fig. 3, VP-SFM supplemented with Hypep 1510 (1.87*10³ PFU/cell) showed an increase in productivity in comparison to VP-SFM with 5% FBS (1.69*10³ PFU/cell).

These results demonstrate that the production of H1-PV in VP-SFM supplemented with Hypep 1510 showed the same yield compared to VP-SFM supplemented with 5% FBS. Thus, the VP-SFM medium of the present invention, when supplemented with Hypep 1510, can be used to culture H-1 PV-infected NB-324K cells in serum-free medium with comparable results as obtained with medium supplemented with 5% FBS.

### Example 5

### Influence of the pH during infection for the H-1 PV productivity

An ideal pH for the H-1 PV production should be determined. Therefore the pH of 6.5, 7.0, 7.2, 7.5, 7.8, 8.0 and 8.5 was adjusted in the MEM media either with HCl or NaOH. To ensure the stable pH during H-1 PV virus infection cell culture bottles with closed lids were used to avoid gas exchange for 4 hours. Because CO₂-gasi is necessary for the cultivation of the cells, after 4 hours incubation lids with filter were used for 4 days incubation. The cells were harvest as described above and the infectious particles have been analyzed via PFU Assay.

As shown in Fig. 4, the pH should be adjusted in the range of 7.0 to 7.5, preferably to about 7.2, to achieve an optional yield of infectious particles.

### Example 6

### Morphology of NB-324K in MEM-Hepes, VP-SFM and Opti-Pro SFM media

As shown in Fig. 5 via light microscopical pictures, the morphology demonstrates the same polygonal shape with regular dimensions and size in all three mediums.

As shown in Fig. 6 via immunofluorescence, the master cell line (MCB) is an epithelial cell line like the compared 293T human embryonic kidney cell line and differs from the fibroblast cell line HEF.

### List of Reference

1. Angelova, A.L., Aprahamian, M., Balboni, G., Delecluse, H.J., Feederle, R., Kiprianova, I., Grekova, S.P., Galabov, A.S., Witzens-Harig, M., Ho, A.D., Rommelaere, J. and Raykov, Z., 2009a. Oncolytic rat parvovirus H-1 PV, a candidate for the treatment of human lymphoma: In vitro and in vivo studies. Molecular therapy; The journal of the American Society of Gene Therapy 17, 1164-72.
2. Angelova, A.L., Aprahamian, M., Grekova, S.P., Hajri, A., Leuchs, B., Giese, N.A., Dinsart, C., Herrmann, A., Balboni, G., Rommelaere, J. and Raykov, Z., 2009b. Improvement of Gemcitabine-Based Therapy of Pancreatic Carcinoma by Means of Oncolytic Parvovirus H-1 PV. Clinical Cancer Research 15, 511-519.
3. Burnett, E., Cotmore, S.F. and Tattersall, P., 2006. Segregation of a single outboard left-end origin is essential for the viability of parvovirus minute virus of mice. J Virol 80, 10879-83.
4. Cotmore, S.F., Agbandje-McKenna, M., Chiorini, J.A., Mukha, D.V., Pintel, D.J., Qiu, J., Soderlund-Venermo, M., Tattersall, P., Tijssen, P., Gatherer, D. and Davison, A.J., 2014. The family Parvoviridae. Archives of virology 159, 1239-47.
5. Dupressoir, T., Vanacker, J.M., Cornelis, J.J., Duponchel, N. and Rommelaere, J., 1989. Inhibition by parvovirus H-1 of the formation of tumors in nude mice and colonies in vitro by transformed human mammary epithelial cells. Cancer research 49, 3203-8.
6. Faisst, S., Faisst, S.R., Dupressoir, T., Plaza, S., Pujol, A., Jauniaux, J.C., Rhode, S.L. and Rommelaere, J., 1995. Isolation of a Fully Infectious Variant of Parvovirus H-1 Supplanting the Standard Strain in Human-Cells. Journal of Virology 69, 4538-4543.
7. Faisst, S., Guittard, D., Benner, A., Cesbron, J.Y., Schlehofer, J.R., Rommelaere, J. and Dupressoir, T., 1998. Dose-dependent regression of HeLa cell-derived tumours in SCID mice after parvovirus H-1 infection. International journal of cancer. Journal international du cancer 75, 584-9.
8. Fallon, A. E., Rozin, P., and Pliner, P., 1984. The child's conception of food: The development of food rejections with special reference to disgust and contamination sensitivity. Child Devel. 55: 566-575.
9. Geletneky, K., Huesing, J., Rommelaere, J., Schlehofer, J.R., Leuchs, B., Dahm, M., Krebs, O., von Knebel Doeberitz, M., Huber, B. and Hajda, J., 2012. Phase I/IIa study of intratumoral/intracerebral or intravenous/intracerebral administration of Parvovirus H-1 (ParvOryx) in patients with progressive primary or recurrent glioblastoma multiforme: ParvOryx01 protocol. BMC cancer 12, 99.
10. Geletneky, K., Kiprianova, I., Ayache, A., Koch, R., Herrero, Y.C.M., Deleu, L., Sommer, C., Thomas, N., Rommelaere, J. and Schlehofer, J.R., 2010. Regression of advanced rat and human gliomas by local or systemic treatment with oncolytic parvovirus H-1 in rat models. Neuro-oncology 12, 804-14.
11. Grekova, S.P., Aprahamian, M., Daeffler, L., Leuchs, B., Angelova, A., Giese, T., Galabov, A., Heller, A., Giese, N.A., Rommelaere, J. and Raykov, Z., 2011. Interferon gamma improves the vaccination potential of oncolytic parvovirus H-1 PV for the treatment of peritoneal carcinomatosis in pancreatic cancer. Cancer biology & therapy 12, 888-95.
12. Griffith, O.M., 2006. Practical Techniques for centrifugal separations. FiberLite, Piramon Technologies, Inc..
13. Halder, S., Nam, H.J., Govindasamy, L., Vogel, M., Dinsart, C., Salome, N., McKenna, R. and Agbandje-McKenna, M., 2012. Production, purification, crystallization and structure determination of H-1 Parvovirus. Acta crystallographica. Section F, Structural biology and crystallization communications 68, 1571-6.
14.Hanson, N.D. and Rhode, S.L., 3rd, 1991. Parvovirus NS1 stimulates P4 expression by interaction with the terminal repeats and through DNA amplification. J Virol 65, 4325-33.
15.Keay, L., 1975. Biotechnol. Bioengign. 17:745-764
16.Kiprianova, I., Thomas, N., Ayache, A., Fischer, M., Leuchs, B., Klein, M., Rommelaere, J. and Schlehofer, J.R., 2011. Regression of Glioma in Rat Models by Intranasal Application of Parvovirus H-1. Clinical Cancer Research 17, 5333-5342.
17.Kongsvik, J.R. and Toolan, H.W., 1972. Effect of proteolytic enzymes on the hemagglutinating property of the parvoviruses, H-1, H-3, and RV. Proceedings of the Society for Experimental Biology and Medicine. Society for Experimental Biology and Medicine 140, 140-4.
18. Lacroix, J., Leuchs, B., Li, J., Hristov, G., Deubzer, H.E., Kulozik, A.E., Rommelaere, J., Schlehofer, J.R. and Witt, O., 2010. Parvovirus H1 selectively induces cytotoxic effects on human neuroblastoma cells. International journal of cancer. Journal international du cancer 127, 1230-9.
19. Li, J., Bonifati, S., Hristov, G., Marttila, T., Valmary-Degano, S., Stanzel, S., Schnolzer, M., Mougin, C., Aprahamian, M., Grekova, S.P., Raykov, Z., Rommelaere, J. and Marchini, A., 2013. Synergistic combination of valproic acid and oncolytic parvovirus H-1 PV as a potential therapy against cervical and pancreatic carcinomas. EMBO molecular medicine 5, 1537-55.
20.Nuesch, J.P., Lacroix, J., Marchini, A. and Rommelaere, J., 2012. Molecular pathways: rodent parvoviruses-mechanisms of oncolysis and prospects for clinical cancer treatment. Clin Cancer Res 18, 3516-23.
21. Paradiso, P.R., 1981. Infectious process of the parvovirus H-1: correlation of protein content, particle density, and viral infectivity. J Virol 39, 800-7.
22. Rommelaere, J., Geletneky, K., Angelova, A.L., Daeffler, L., Dinsart, C., Kiprianova, I., Schlehofer, J.R. and Raykov, Z., 2010. Oncolytic parvoviruses as cancer therapeutics. Cytokine & growth factor reviews 21, 185-95.
23.Toolan, H.W., Dalldore, G., Barclay, M., Chandra, S. and Moore, A.E., 1960. An Unidentified, Filtrable Agent Isolated from Transplanted Human Tumors. Proceedings of the National Academy of Sciences of the United States of America 46, 1256-8.
24.Werner, R. G. et al, Mammalian Cell Cultures Part I: Characterization, morphology and metabolism, in: Arzneim.-Forsch./Drug Res. 43:1134-1139 (1993)).
25. Wrzesinski, C., L. Tesfay, N. Salome, J. C. Jauniaux, J. Rommelaere, J. Cornelis, and C. Dinsart. 2003. Chimeric and pseudotyped parvoviruses minimize the contamination of recombinant stocks with replication-competent viruses and identify a DNA sequence that restricts parvovirus H-1 in mouse cells. J. Virol. 77:3851-3858.)
26.Zolotukhin, S., Byrne, B.J., Mason, E., Zolotukhin, I., Potter, M., Chesnut, K., Summerford, C., Samulski, R.J. and Muzyczka, N., 1999. Recombinant adeno-associated virus purification using novel methods improves infectious titer and yield. Gene Ther 6, 973-85.
27. UK Patent Application No. GB 901676

## Claims

1. A method for growing the producer cell line NB-324K infected with parvovirus H-1, comprising
culturing the infected NB-324K cells in a serum-free medium comprising 16-22 mM glucose, 3-5 mM glutamine, 0.1-0.6 mM glutamate, 0.5-1.0 mM lactate, less than 0.3 mM ammonium and 3-10 µg/µl proteins, wherein the medium has a pH of 7.0 - 7.5.

2. The method according to claim 1, wherein said medium contains 19.14 mM glucose, 4.25 mM glutamine, 0.174 mM glutamate, 0.669 mM lactate, less than 0.05 mM ammonium and about 5 µg/µl proteins comprising insulin, epithelial growth factor and vegetables components.

3. The method of claim 2, wherein the vegetable component is based on soy peptides.

4. The method according to claim 1, wherein said medium contains 17.49 mM glucose, 4.25 mM glutamine, 0.486 mM glutamate, 0.641 lactate, 0.18 mM ammonium and 7.5 µg/µl epithelial growth factor.

5. The method according to claim 3, wherein said soy peptide is Hypep 1510 ®.

6. The method according to any one of claim 1 to 4, wherein the pH is about 7.2.
